# DEMANDE DE BREVET EUROPEEN

(11) **EP 1 018 540 A1**
(43) Date de publication de la demande: **12.07.2000**
(21) Numéro de dépôt: 00430001.8
(22) Date de dépôt: 07.01.2000
(51) Int. Cl.: C11C 3/00, A61K 7/48

(54) **Procédé de fabrication d'un émollient non gras à partir d'extraits d'olives**

(30) Priorité: 08.01.1999 FR 9900252
(71) Demandeur: Sophim, 04310 Peyruis (FR)
(72) Inventeur: Cecchi, Gorges, 13010 Marseille (FR)
(74) Mandataire: Domange, Maxime

(57) **Abrégé**

L'invention concerne un procédé d'obtention d'un émollient non gras d'origine végétale, qui comprend les étapes suivantes :
a) l'estérification des acides gras libres de condensats de raffinage physique d'huile d'olive enrichis en squalène avec un alcool gras d'origine végétale, en présence d'un catalyseur;
b) la neutralisation des acides gras libres n'ayant pas réagi par une base et la récupération du produit d'estérification ;
c) la distillation du produit d'estérification ;
d) la désodorisation du distillat obtenu ;
e) la décoloration du produit désodorisé ; et
f) la frigélisation du produit décoloré, puis la filtration du précipité formé pour récupérer le filtrat.

## Description

La présente invention est relative à un procédé de fabrication concernant un émollient non gras, d'origine entièrement végétale. Le domaine technique de l'invention est celui de la chimie fine.

Les émollients sont abondamment utilisés en cosmétique et en pharmacie, pour rendre souple une peau sèche et pour en améliorer son élasticité. Le terme émollient désigne généralement un ensemble de perceptions transmises par le toucher et par la vue. Les perceptions induites par le toucher évoquent la douceur, l'élasticité et le pouvoir glissant. Les perceptions induites par la vue évoquent le brillant et le mat.

Le nombre des émollients proposés par les fournisseurs de matières premières cosmétiques est considérable. Ces émollients se distinguent les uns des autres par leur nature chimique mais également par la résultante de deux grandeurs : l'émollience à l'application et l'émollience résiduelle. Il existe ainsi des émollients à effet protecteur, d'autres à effet surgraissant, certains donnent l'impression d'un effet sec, d'autres enfin agissent comme des astringents.

La grande majorité de ces émollients est caractérisée par la présence d'acides gras à chaîne carbonée plus ou moins longue, linéaire ou ramifiée. Ces acides gras sont eux-mêmes combinés sous forme d'esters, à des alcools à chaîne carbonée plus ou moins longue, linéaire ou ramifiée. Ce sont ces esters et leurs acides gras qui constituent la base de l'effet d'émollience. On considère de manière générale qu'il existe dans cette catégorie d'émollient deux familles d'esters : ceux ayant une origine totalement naturelle, ceux ayant une origine synthétique, la synthèse impliquant l'estérification de l'acide gras par l'alcool. Les esters de synthèse sont généralement fabriqués à partir d'acides gras saturés, ce qui leur confère une grande stabilité vis à vis de l'oxydation, mais leur enlève toute possibilité de jouer un rôle par intégration dans les processus biosynthétiques se développant au niveau de l'épiderme. Il est en effet bien connu que les acides gras polyinsaturés (linoléique et linolénique) dits acides gras essentiels peuvent se transformer sous l'effet des lipases que contient l'épiderme, en d'autres acides gras polyinsaturés susceptibles de limiter la perte d'eau transépidermique. C'est cet effet d'émollience particulier que l'on recherche dans la deuxième catégorie d'esters d'origine naturelle. C'est dans celle catégorie que l'on peut classer les huiles végétales à l'état vierge (ou brutes) ou raffinées.

Par définition les huiles végétales se classent dans la grande famille des corps gras. Ces derniers sont constitués par un mélange d'esters qui sont des triacyl-glycérols ou triesters de glycérol et d'acides gras. C'est la nature des acides gras impliqués dans ces esters qui va donner sa consistance au corps gras résultant de leur mélange. Ainsi plus la composition de ces corps gras sera riche en acides gras saturés et plus leur consistance sera élevée au point d'obtenir des graisses ou des beurres, plus ou moins concrètes à 20°C. Inversement, plus la teneur en acides gras (mono- et poly-) insaturés sera importante, plus le corps gras aura tendance à être totalement fluide à 20°C, ce qui justifie son appellation d'huile.

C'est le cas des huiles végétales, caractérisées par une composition en acides gras dont la teneur globale en acides gras insaturés est souvent supérieure à 85 %. La consistance liquide des huiles est un premier avantage qui va dans le sens de l'obtention d'un effet émollient. Il faut ajouter à l'effet de la consistance liquide celui des acides gras essentiels comme l'acide linoléique toujours présent dans les huiles végétales à des teneurs variables qui sont fonction de l'origine botanique des espèces oléagineuses dont elles sont issues. Comme nous l'avons déjà signalé précédemment, la transformation de cet acide linoléique en d'autres acides gras insaturés via un processus biosynthétique, se traduit par un effet hydratant important contribuant à maintenir l'épiderme dans un bon état d'émollience. Il faut enfin tenir compte pour les huiles végétales de l'effet biologique important joué par les composants mineurs de leur partie insaponifiable tels que le squalène, les carotènes, les alcools triterpènique et les phytostérols.

Si ces trois avantages ont bien été mis en évidence il n'en demeure pas moins que les huiles végétales présentent le grave désavantage de donner un toucher gras après leur application sur la peau en raison de leur faible vitesse de pénétration dans l'épiderme. On reconnaît de manière générale que la vitesse de pénétration percutanée d'une molécule est inversement proportionnelle à son poids moléculaire. Cette vitesse est optimum pour un poids moléculaire de 400 Dalton. Or le poids moléculaire des triglycérides des huiles végétales est centré autour de 870 Dalton. On comprendra donc facilement que les huiles végétales telle que celles utilisées dans les formulations cosmétiques et pharmaceutiques puissent laisser cette impression de gras donnée par les triglycérides restant sur la peau.

A ce désavantage incontournable lié à l'utilisation des huiles végétales comme émollient, il faut ajouter l'absence totale d'"effet coussin" de ces dernières. L'effet coussin peut se définir comme un effet d'assouplissement externe de l'épiderme donnant une impression de moelleux rappelant celui produit par un coussin. Cet effet est dû à une substance qui dépose un film non occlusif qui ne pénètre pas instantanément dans l'épiderme, dans la mesure où elle ne présente pas d'affinité pour la couche cornée. Pour obtenir une manifestation de cet effet il faut habituellement rajouter des produits à base de silicones dont l'utilisation en formulation cosmétique devient de plus en plus discutée en raison des problèmes rencontrés par l'introduction de tels composés dans l'organisme.

Le problème posé est donc de procurer des procédés de fabrication d'un émollient d'origine entièrement végétale, liquide à 20 °C, dont le poids moléculaire des composants principaux soit situé autour de 400 Dalton, qui permette d'obtenir une préparation émolliente à toucher non gras; un autre objectif de l'invention est également de procurer une préparation émolliente contenant suffisamment d'acide linoléique précurseur d'effet hydratant sans que sa teneur soit trop élevée pour ne pas trop favoriser un effet pro-oxydant; le dernier objectif de l'invention consiste à obtenir une préparation manifestant un "effet coussin" élevé afin de limiter ou d'éviter l'introduction de produits à base de silicones dans les formulations cosmétiques.

Ce problème est résolu par le procédé selon l'invention, qui comprend les étapes suivantes:
a) l'estérification des acides gras libres de condensats de raffinage physique d'huile d'olive enrichis en squalène avec un alcool gras d'origine végétale, en présence d'un catalyseur;
b) la neutralisation des acides gras libres n'ayant pas réagi par une base et la récupération du produit d'estérification ;
c) la distillation du produit d'estérification ;
d) la désodorisation du distillat obtenu;
e) la décoloration du produit désodorisé ; et
f) la frigélisation du produit décoloré, puis la filtration du précipité formé pour récupérer le filtrat.

Avantageusement dans la matière première de départ, la teneur en poids des acides gras libres par rapport au poids des condensats de raffinage d'huile d'olive est au moins égale à 50 % et de préférence supérieure à 70 %, de même que la teneur en poids de squalène par rapport au poids des condensats de raffinage d'huile d'olive est au moins égale à 5 % et de préférence supérieure à 10 %.

Les condensats de raffinage physique d'huile d'olive sont obtenus, selon des méthodes bien connues de l'homme du métier, par exemple par condensation des effluents provenant de l'élimination des acides gras libres d'une huile d'olive lampante (huile trop acide qui doit être raffinée). Cette élimination se fait par distillation dite 〈〈 neutralisante 〉〉 des acides gras libres dans des conditions où ils sont volatils (température 240°C - pression 2 mbar - entraînement à la vapeur d'eau). Cette distillation entraîne non seulement les acides gras libres, mais aussi celle des composants de l'insaponifiable de l'huile d'olive dont notamment le squalène.

L'alcool gras d'origine végétale utilisé dans l'étape d'estérification peut notamment être choisi parmi les alcanols en C₁-C₂₀, les alcénols en C₃-C₂₀ ou les alcools ramifiés en C₃-C₂₀. Ces alcools ramifiés sont des alcools susceptibles de porter des substituants alkyle en C₁-C₈. Parmi les alcanols en C₁-C₂₀, on choisit de préférence ceux en C₄-C₁₂, en particulier ceux en C₆-C₁₀ ; parmi les alcools ramifiés en C₃-C₂₀, on choisit de préférence ceux en C₈-C₂₀.

Selon un mode de réalisation préféré de l'invention, on choisit l'alcool gras d'origine végétale parmi le 1-décanol, le 1-octanol, le 1-hexanol, l'alcool oléique ou l'hexyldécanol.

Le catalyseur utilisé à l'étape a) est généralement une lipase, un acide fort, un acide de Lewis ou une base alcaline.

Avantageusement, ce catalyseur est choisi parmi la lipase de Mucor Miehei supportée, la soude, l'acide toluène-4-sulfonique ou le zinc en poudre.

De préférence, l'estérification des acides gras libres desdits condensats de raffinage d'huile d'olive par lesdits alcools, en présence desdits catalyseurs, est faite sous agitation, et l'agitation dure au plus dix heures et, de préférence, au moins 3 heures ; l'estérification est réalisée sous vide, au moins égal à 80 mbar et au plus égal à 15 mbar ; et l'estérification est réalisée à une température au moins égale à 40 °C et au plus égale à 220 °C.

Avantageusement, la neutralisation des acides gras libres résiduels est réalisée à l'étape b) avec une quantité stoechiométrique de soude ou potasse par rapport auxdits acides gras libres, en solution aqueuse au moins N et au plus 2N, par agitation à température ambiante pendant une durée au moins égale à une demi-heure et au plus égale à une heure, les savons formés étant éliminés après l'arrêt de l'agitation par décantation.

De préférence, la distillation du produit neutralisé est faite à l'étape c) sous un vide au moins égal à 1 mbar et au plus égal à 0,1 mbar, et à une température au moins égale à 180 °C et au plus égale à 260 °C, pendant une durée au plus égale à 4 heures et de préférence égale à 2 heures.

Généralement, la désodorisation du distillat obtenu est faite à l'étape d) sous un vide au moins égal à 4 mbar et au plus égal à 1 mbar, et à une température au moins égale à 200 °C et au plus à 220 °C. Ladite opération de désodorisation est de préférence effectuée avec une quantité de vapeur d'eau au moins égale à 5 % en poids et au plus à 15 % en poids de la quantité de produit à désodoriser, pendant une durée au moins égale à 1,5 heures, et au plus égale à 3 heures.

Avantageusement, la décoloration est faite à l'étape e) par percolation sur colonne de gel de silice du produit désodorisé, avec une proportion de gel de silice au moins égale à 5 % en poids et au plus égale à 15 % en poids de la quantité de produit à décolorer, et est faite à température et pression normales.

De préférence, la frigélisation est faite à l'étape f) par agitation du produit décoloré, à une température au moins égale à 10°C et au plus égale à 14°C, et est poursuivie pendant une durée au moins égale à 1 heure et au plus égale à 4 heures, puis le produit est filtré.

Avantageusement, le filtrat obtenu a une teneur en squalène au moins égale à 5 % et de préférence au moins égale à 10 %, ainsi qu'une teneur en esters d'acides gras (cires-ester) au moins égale à 85 % et au plus égale à 93%.

Selon un autre aspect, l'invention concerne un émollient non gras susceptible d'être obtenu par le procédé décrit ci-dessus. Cet émollient non-gras présente les caractéristiques cosmétiques suivantes:
- liquide à 20 °C,
- parfaitement compatible avec l'épiderme,
- "effet coussin" important,
- toucher sec et soyeux,
- grand pouvoir d'étalement,
- pénétration rapide dans l'épiderme.

L'invention sera mieux comprise à l'aide des exemples ci-après, donnés à titre purement illustratif.

### Exemple 1

On verse 756 g de condensats de raffinage physique d'huile d'olive contenant 71 % d'acides gras libres et 8 % de squalène dans un ballon à une tubulure, lesdits condensats de raffinage ayant été au préalable fondus par simple chauffage extérieur du récipient qui les contient. On verse ensuite dans ledit ballon 530 g d'alcool oléique ainsi que 19 g de Lipozyme (enzyme supportée vendue par la société NOVO-NORDISK). Après création du vide dans le ballon, on chauffe à la température de 65 °C et on agite le contenu du ballon de façon à mettre en suspension l'enzyme. Lorsque la majeure partie des acides gras libres a été estérifiée, l'enzyme est séparée par simple décantation au fond du ballon. On recueille 1228 g de liquide surnageant de couleur marron possédant toujours la forte odeur des condensats de raffinage d'huile d'olive, avec une teneur en cires-ester de 76,9%, une teneur en squalène de 4,8 %, une teneur en acides gras libres de 1,4% (exprimée en acide oléique) et une teneur en alcool oléique de 3,6 %.

### Exemple 2

On verse 756 g de condensats de raffinage physique d'huile d'olive contenant 71 % d'acides gras libres et 8 % de squalène dans un ballon à une tubulure, lesdits condensats ayant été au préalable fondus par simple chauffage extérieur du récipient qui les contient. On verse ensuite dans ledit ballon 257 g de 1-octanol ainsi que 2,3 g de solution aqueuse de soude à 50%. Après création du vide dans le ballon, on agite le mélange réactionnel et la température est progressivement montée à 200 °C en 0,5 heure. Après 9 heures de réaction dans ces conditions on a estérifié la majeure partie des acides gras libres. On recueille 975 g d'un liquide homogène de couleur marron possédant toujours la forte odeur caractéristique des condensats de raffinage d'huile d'olive, avec une teneur en cires-ester de 72,7 %, une teneur en squalène de 6,2 %, une teneur en acides gras libres de 1,9 % (exprimé en acide oléique) et une teneur en 1-octanol de 2,2 %.

### Exemple 3

On verse 756 g de condensats de raffinage physique d'huile d'olive contenant 71 % d'acides gras libres et 8 % de squalène dans un ballon à une tubulure, lesdits condensats ayant été au préalable fondus par simple chauffage extérieur du récipient qui les contient. On verse ensuite 202 g de 1-hexanol ainsi que 3,8 g de zinc en poudre (réf. Fluka N^{o} 96454). Après création du vide dans le ballon, on agite le mélange réactionnel et la température est progressivement montée à 200 °C en 0,5 heure. Après 6 heures de réaction dans ces conditions on a estérifié la majeure partie des acides gras libres. On recueille 902 g d'un liquide homogène de couleur marron possédant toujours la forte odeur caractéristique des condensats de raffinage d'huile d'olive, avec une teneur en cires-ester de 73,3 %, une teneur en squalène de 6,7 %, une teneur en acides gras libres de 0,4 % (exprimée en acide oléique) et une teneur en 1-hexanol de 1,3 %.

### Exemple 4

On verse 756 g de condensats de raffinage physique d'huile d'olive contenant 71 % d'acides gras libres et 8 % de squalène dans un ballon à une tubulure, lesdits condensats ayant été au préalable fondus par simple chauffage extérieur du récipient qui les contient. On verse ensuite 495 g d'hexyldécanol ainsi que 3,8 g d'acide toluène-4-sulfonique (réf. Fluka N° 89762). Après création du vide dans le ballon, on agite le mélange réactionnel et la température est progressivement montée à 200 °C en 0,5 heure. Après 3 heures de réaction dans ces conditions on a estérifié la majeure partie des acides gras libres. On recueille 1191 g d'un liquide homogène de couleur marron possédant toujours la forte odeur caractéristique des condensats de raffinage d'huile d'olive, avec une teneur en cires-ester de 77,9 %, une teneur en squalène de 5,0 %, une teneur en acides gras libres de 0,6 % (exprimée en acide oléique) et une teneur en hexyldécanol de 2,5 %.

### Exemple 5

On verse 756 g de condensats de raffinage physique d'huile d'olive contenant 71 % d'acides gras libres et 8 % de squalène dans un ballon à une tubulure, lesdits condensats de raffinage ayant été au préalable fondus par simple chauffage extérieur du récipient qui les contient. On verse ensuite dans ledit ballon 316 g de 1-décanol ainsi que 20 g de Lipozyme. Après création du vide dans le ballon, on chauffe le mélange réactionnel à 65° C sous agitation. Lorsque la majeure partie des acides gras libres a été estérifiée, l'enzyme est séparée du produit obtenu par simple décantation au fond du ballon. On recueille 1015 g de liquide surnageant de couleur marron et possédant toujours la forte odeur des condensats de raffinage d'huile d'olive, avec une teneur en cires-ester de 73,9%, une teneur en squalène de 5,8 %, une teneur en acides gras libres de 1,7% (exprimée en acide oléique) et une teneur en 1-décanol de 2,6 %.

### Exemple 6

Le produit d'estérification obtenu à l'exemple 5 est soumis à une neutralisation par la potasse aqueuse 2N dans les conditions suivantes: On ajoute régulièrement en 10 minutes sous agitation, 30 ml de la solution aqueuse de potasse 2 N à 1000 g dudit produit d'estérification. Le système est laissé sous agitation pendant 30 minutes, puis on laisse décanter les savons et l'eau. La phase inférieure est écartée et l'on récupère 939 g d'un produit liquide homogène ayant les mêmes caractéristiques de couleur et d'odeur qu'avant neutralisation avec une acidité libre de 0,2 %. 930 g dudit liquide homogène sont distillés sous vide (1 mbar) et sous microbullage d'azote, en chauffant progressivement le ballon de façon à ce que la température atteinte par le liquide résiduel en fin de distillation ne dépasse pas 260 °C. Le vide en cours de distillation se situe autour de 0,6 mbar. La distillation est arrêtée au bout de 2 heures alors qu'il ne reste plus au fond du ballon que des triglycérides non volatils dans ces conditions. On récupère dans le ballon 788 g d'un distillat constitué d'un produit liquide à température ambiante, de couleur jaune foncé, ayant une odeur identique à celle du produit avant distillation. Ledit distillat est alors soumis à une désodorisation qui est réalisée dans les conditions suivantes : 780 g dudit distillat sont placés dans un ballon à 3 tubulures relié à un système de distillation. Après mise sous vide et admission de la vapeur, on chauffe progressivement en stabilisant la température à 210 °C et la pression à 2 mbar. Après 2 heures de désodorisation et passage de 75 g de vapeur, le chauffage est coupé, de même que l'admission de vapeur, et le ballon refroidi. On récupère 705 g d'un liquide jaune foncé ayant perdu une grande partie de son odeur, qui contient 7,0 % de squalène et 91,6 % de cires-ester, qui va être soumis à une décoloration, réalisée par passage sur colonne de silice. Pour cela on fait percoler 700 g dudit liquide obtenu par désodorisation sur une colonne de gel de silice (réf. Merck N° 7734). La percolation desdits 700 g permet d'obtenir 660 g d'un produit liquide jaune clair, pratiquement sans odeur, contenant 7,0 % de squalène et 92 % de cires-ester. Ce produit liquide laisse apparaître après repos à 20 °C un léger précipité qui a tendance à décanter. Il est alors nécessaire pour éviter que ce trouble n'apparaisse à une température supérieure à 15°C de procéder à sa frigélisation. 650 g dudit produit percolé sur silice sont introduits dans un réacteur cylindrique de 2 litres équipé d'une double enveloppe extérieure permettant le passage d'un fluide refroidissant. Ledit liquide est progressivement refroidi à une température de 14,5 °C, par passage du liquide refroidissant porté lui-même à 14 °C dans la double enveloppe dudit réacteur de frigélisation, sous agitation pendant 3 heures, puis il est filtré. Le liquide obtenu de couleur jaune clair, sans odeur marquée, parfaitement limpide, contient 7,0 % de squalène et 92 % de cires-ester.

La composition en acides gras du produit obtenu s'établit ainsi:
- Acide palmitique: : 18,3 %
- Acide palmitoléique: : 1,1 %
- Acide stéarique: : 3,5 %
- Acide oléique: : 66,6 %
- Acide linoléique: :10,5 %

## Revendications

1. Procédé d'obtention d'un émollient non gras d'origine végétale, qui comprend les étapes suivantes :
a) l'estérification des acides gras libres de condensats de raffinage physique d'huile d'olive enrichis en squalène avec un alcool gras d'origine végétale, en présence d'un catalyseur ;
b) la neutralisation des acides gras libres n'ayant pas réagi par une base et la récupération du produit d'estérification ;
c) la distillation du produit d'estérification ;
d) la désodorisation du distillat obtenu ;
e) la décoloration du produit désodorisé ; et
f) la frigélisation du produit décoloré, puis la filtration du précipité formé pour récupérer le filtrat.

2. Procédé selon la revendication 1, dans lequel à l'étape a) la teneur en acides gras libres est au moins égale à 50 % en poids, et la teneur en squalène est au moins égale à 5 % en poids, par rapport au poids des condensats.

3. Procédé selon la revendication 1 ou 2, dans lequel l'alcool gras est choisi parmi les alcanols en C₁-C₂₀, les alcénols en C₃-C₂₀, ou les alcools ramifiés en C₃-C₂₀.

4. Procédé selon la revendication 3, dans lequel l'alcool gras est choisi parmi le 1-hexanol, le 1-octanol, le 1-décanol, l'hexyldécanol ou l'alcool oléique.

5. Procédé selon l'une des revendications 1 à 4, dans lequel le catalyseur est choisi parmi les lipases, les acides forts, les acides de Lewis ou les bases alcalines.

6. Procédé selon la revendication 5, dans lequel le catalyseur est choisi parmi la lipase de Mucor Miehei supportée, la soude, l'acide toluène-4-sulfonique ou le zinc en poudre.

7. Procédé selon l'une des revendications 1 à 6, dans lequel la réaction d'estérification est réalisée à une température comprise entre 40 et 220°C.

8. Procédé selon l'une des revendications 1 à 7, dans lequel la distillation du produit d'estérification est réalisée à une température comprise entre 180 et 260°C, sous atmosphère inerte.

9. Procédé selon l'une des revendications 1 à 8, dans lequel la désodorisation est effectuée avec une quantité de vapeur d'eau comprise entre 5 et 15 % en poids du distillat obtenu à l'étape c).

10. Procédé selon l'une des revendications 1 à 9, dans lequel la frigélisation du produit décoloré est effectuée à une température au moins égale à 10°C et au plus égale à 14°C.

11. Procédé selon l'une des revendications 1 à 10, dans lequel le filtrat obtenu à l'étape f) a une teneur en esters d'acides gras au moins égale à 85 % en poids et une teneur en squalène au moins égale à 5 % en poids.

12. Emollient non gras d'origine végétale susceptible d'être obtenu par le procédé selon l'une des revendications 1 à 11.
